# FASCICULE DE BREVET EUROPEEN

(11) **EP 1 123 406 B1**
(45) Date de publication et mention de la délivrance du brevet: **26.07.2006**
(21) Numéro de dépôt: 99952734.4
(22) Date de dépôt: 20.10.1999
(51) Int. Cl.: C12N 15/82, C12N 9/02, C12N 5/10, G01N 33/50, A01H 5/00

(54) **SEQUENCE D'ADNc TRANSCRIVANT UN ARNm CODANT POUR L'OXYDASE TERMINALE ASSOCIEE A LA BIOSYNTHESE DES CAROTENOIDES ET UTILISATIONS**
CDNA-SEQUENZ, WELCHE FÜR DIE MIT DER CAROTINOIDBIOSYNTHESE ASSOZIERTE TERMINALE OXIDASE KODIERT UND DEREN ANWENDUNGEN
cDNA SEQUENCE TRANSCRIBING A mRNA CODING FOR THE TERMINAL OXYDASE ASSOCIATED WITH CAROTENOID BIOSYNTHESIS AND USES

(30) Priorité: 20.10.1998 FR 9813283
(43) Date de publication de la demande: 16.08.2001
(73) Titulaire: UNIVERSITE JOSEPH FOURIER, 38041 Grenoble Cédex 9 (FR)
(72) Inventeur: CAROL, Pierre, Uni. Joseph Fourier, F-38041 Grenoble Cedex (FR); KUNTZ, Marcel, Uni. Joseph Fourier, F-38041 Grenoble Cedex (FR); MACHE, Régis, Uni. Joseph Fourier, F-38041 Grenoble Cedex (FR)
(74) Mandataire: Guerre, Dominique
(86) Numéro de dépôt international: PCT/IB1999/001719
(87) Numéro de publication internationale: WO 2000/023605

(56) Documents cités:
- WO-A-91/09128
- WO-A-95/34668
- WETZEL, C.M., ET AL.: "nulcear-organelle interactions: the immutans variegation mutant of Arabidopsis is plastid autonomous and impaired in carotenoid biosynthesis" THE PLANT JOURNAL, vol. 6, no. 2, 1994, pages 161-175, XP002110646 cité dans la demande
- NEWMAN, T., ET AL.: "Genes galore: a summary of methods for accessing results from large-scale partial sequencing of anonymous Arabidopsis cDNA clones" EMBL SEQUENCE DATA LIBRARY, 3 février 1995 (1995-02-03), XP002110647 heidelberg, germany
- BEVAN, M., ET AL.: "Arabidopsis thaliana chromosome 4, BAC clone T10I14" EMBL SEQUENCE DATA LIBRARY, 3 février 1998 (1998-02-03), XP002110648
- BEVAN, M., ET AL.: "hypothetical 38.7 KD protein" EMBL SEQUENCE DATA LIBRARY, 1 juin 1998 (1998-06-01), XP002110649 heidelberg, germany
- FINNEGAN, P.M., ET AL.: "untitled" EMBL SEQUENCE DATA LIBRARY, 15 juillet 1998 (1998-07-15), XP002110650 heidelberg, germany
- FINNEGAN, P.M., ET AL. : "differential expression of the multigene family encoding the soybean mitochondrial alternative oxidase" PLANT PHYSIOLOGY, vol. 114, no. 2, juin 1997 (1997-06), pages 455-466, XP002131002
- CAROL, P., ET AL.: "mutations in the Arabidopsis gene IMMUTANS cause a variegated phenotype by inactivating a chloroplast terminal oxidase associated with Phytoene desaturation" THE PLANT CELL, vol. 11, janvier 1999 (1999-01), pages 57-68, XP002110651
- WU, D., ET AL.: "THE IMMUTANS VARIEGATION LOCUS OF ARABIDOPSIS DEFINES A MITOCHONDRIAL ALTERNATIVE OXIDASE HOMOLOG THAT FUNCTIONS DURING EARLY CHLOROPLAST BIOGENESIS" THE PLANT CELL, vol. 11, janvier 1999 (1999-01), pages 43-55, XP002131003
- SMITH, H.: "photosynthetic pigmentation - variegations on a theme" THE PLANT CELL, vol. 11, 1 janvier 1999 (1999-01-01), pages 1-3, XP002131004

## Description

L'invention concerne une séquence d'ADN (acide désoxyribonucléique) décrite par SEQ ID NO:1, transcrivant un ARNm (acide désoxyribonucléique messager), lui-même codant pour l'enzyme OTBC (Oxydase Terminale associée à la Biosynthèse des Caroténoïdes) décrite par SEQ ID NO:2, ainsi que des vecteurs de transformation de cellule, de plante ou fragment de plante, et le procédé pour modifier la production de caroténoïdes dans une plante.

Les caroténoïdes sont des pigments lipophiles synthétisés chez les plantes, les champignons et les bactéries. Dans les tissus photosynthétiques, les caroténoïdes ont une fonction de pigment accessoire d'absorption de la lumière et surtout de photoprotection contre les radicaux libres, tels que l'oxygène singulet.

Chez les plantes et certains micro-organismes, la voie biosynthétique des caroténoïdes produit des carotènes, des xanthophylles et leurs dérivés. Ces composés sont synthétisés à partir du phytoène qui est modifié par des réactions de déshydrogénation successives pour produire du phytofluène, du zéta-carotène, du neurosporène puis du lycopène. Le lycopène s'accumule dans certain cas, donnant par exemple le pigment rouge de la tomate, ou plus généralement se retrouve sous forme modifiée par cyclisation, pour former de l'alpha- ou du béta-carotène. Ces caroténoïdes cyclisés sont les précurseurs de la vitamine A, et peuvent s'accumuler ou donner, par des réactions d'oxydation, les xanthophylles, qui sont des pigments jaunes, roses, oranges ou rouges.
Les étapes de déshydrogénation successives du phytoène sont catalysées chez la plupart des micro-organismes par une enzyme unique appelée phytoène désaturase CRTI. Chez les plantes et les cyanobactéries, deux enzymes apparentées existent. La première, appelée phytoène désaturase (PDS), catalyse la conversion du phytoène en phytofluène puis en zéta-carotène. La seconde,appelée zéta-carotène désaturase (ZDS), catalyse la conversion du zêta-carotène en neurosporène puis en lycopène. Chacune de ces réactions de déshydrogénation nécessite le transfert de deux électrons et deux protons du substrat vers un accepteur. Ces réactions de déshydrogénation nécessitent donc des enzymes, dites structurales, et des cofacteurs, qui sont des intermédiaires dans les réactions d'oxydoréduction.

Les inventeurs de la présente invention ont découvert un nouveau gène codant pour une enzyme appelée OTBC (Oxydase Terminale associée à la Biosynthèse des Caroténoïdes), impliquée dans la biosynthèse des caroténoïdes. Il semble que cette enzyme soit placée dans les membranes des chloroplastes et soit indispensable au bon fonctionnement de la PDS.

Un premier objet selon l'invention concerne donc une séquence d'ADN, comprenant au moins une région codante constituée par :
- la séquence nucléotidique représentée par SEQ ID NO :1, transcrivant un ARNm, cet ARNm codant pour l'enzyme OTBC (Oxydase Terminale associée à la Biosynthèse des Caroténoïdes) décrite par SEQ ID NO: 2, ou son complémentaire
- toute séquence nucléotidique modifiée présentant, par rapport à SEQ ID NO:1, une ou des modifications choisies parmi les additions, les suppressions et les substitutions de nucléotides, cette séquence modifiée présentant avec SEQ ID NO: 1 au moins 70% d'identité et transcrivant un ARNm lui-même codant pour l'OTBC décrite par SEQ ID NO:2, ou codant pour une protéine modifiée de ladite OTBC, ladite protéine modifiée ayant une activité enzymatique équivalente à celle de l'OTBC représentée par SEQ ID NO:2, ou son complémentaire.

En particulier, l'invention concerne les séquences codantes de l'OTBC de la tomate, identifiée par SEQ ID NO :3, et du poivron, identifiée par SEQ ID NO :4, respectivement, et toute séquence dérivée obtenue par modification de ces séquences.

Le gène codant pour l'OTBC est une double hélice d'ADN, comprenant des introns et des exons. La séquence SEQ ID NO:1 est le brin complémentaire (sans les introns) ou ADNc, correspondant au brin d'ADN transcrivant l'ARNm codant pour l'OTBC.

Par activité enzymatique équivalente, on entend que l'enzyme, bien que pouvant être modifiée structurellement dans certaines de ses parties, est néanmoins capable de modifier son substrat. Son activité est sensiblement la même que celle de l'enzyme native. On comprendra que cette enzyme ne peut pas être modifiée au niveau de son site actif. De ce fait, toute modification apportée à la séquence native, par addition, suppression ou substitution d'un ou plusieurs acides aminés, s'entend comme engendrant une activité enzymatique équivalente dans la mesure où l'activité de la protéine native n'est pas affectée par ces modifications.

Par ADN, on peut entendre ADN complémentaire (ou ADNC), c'est à dire la copie de l'ARNm sous sa forme d'ADN grâce à l'action d'une transcriptase inverse. L'ADNc ne comprend pas les introns des séquences d'ADN.

On entend par « capable de s'apparier » dans la présente invention, le fait que dans des conditions d'hybridations données, les séquences nucléotidiques complémentaires s'apparient. L'homme du métier connaît bien, selon les conditions d'hybridation utilisées, quel est le pourcentage d'identité que les séquences doivent présenter pour qu'un appariement ou une hybridation puisse se réaliser. Les conditions de stringence pour obtenir un appariement de séquences voisines sont par exemple une hybridation dans 50% de formamide à 35°C. Pour ce qui concerne les conditions d'hybridation, on se référera notamment à l'article « Molecular Cloning, a laboratory manual, second edition, Sambrook, Fritch & Maniatis, 1989. Cold Spring Harbor Laboratory Press. Cold Spring Harbor, New York, USA ».

On entend par « séquence nucléotidique modifiée» dans la présente invention, toute séquence nucléotidique présentant avec la séquence de référence un degré d'identité inférieur à 100%.

Dans un mode de réalisation préféré selon l'invention, les séquences, nucléotidiques modifiées selon la présente invention présentent au moins 80% d'identité avec la séquence nucléotidique représentée par SEQ ID NO:1, ou de sa séquence complémentaire.

On entend par « identité de nucléotide », la comparaison, lorsque les deux brins sont alignés , de la séquence des nucléotides identiques présents sur les deux brins. On obtient de ce fait, en ramenant au nombre de nucléotides totaux, le pourcentage de nucléotides identiques, c'est à dire l'identité de nucléotide.

Un second objet selon l'invention concerne un ARNm transcrit à partir de la séquence d'ADN selon la définition du premier objet, ou à partir de son complémentaire.

Par « ARNm anti-sens », on entend une séquence d'ARN qui est complémentaire d'une séquence de bases d'un ARNm correspondant, complémentaire dans le sens où chaque base (ou la majorité des bases) dans la séquence anti-sens (se lisant dans le sens 3' vers 5') est capable de s'apparier avec la base correspondante (G avec C, A avec U) dans la séquence d'ARNm se lisant dans le sens 5' vers 3' .

Un troisième objet selon l'invention concerne une protéine ayant l'activité de l'enzyme OTBC décrite par SEQ ID NO:2, et codée par une séquence d'ADN telle que définie précédemment.

Un quatrième objet selon l'invention est un ADN recombiné comprenant une séquence d'ADN définie dans le premier objet selon l'invention, ladite séquence étant insérée dans une séquence hétérologue, lesdites séquences transcrivant tout ou partie d'une séquence ARNm codant pour tout ou partie de l'enzyme OTBC, cette dernière ayant une activité enzymatique équivalente à l'enzyme OTBC de la plante.

Par « séquence hétérologue » on entend, selon la présente invention, toute séquence pouvant être coupée par des enzymes, et servant de ce fait à insérer d'autres séquences présentant des activités diverses.

Avantageusement, l'ADN recombiné défini ci-dessus comprend les éléments nécessaires pour contrôler l'expression de la séquence insérée, particulièrement une séquence promotrice et une séquence de terminaison de transcription desdites séquences.

Un cinquième objet selon l'invention concerne un vecteur de transformation de plantes, adapté pour augmenter la biosynthèse des caroténoïdes, comprenant tout ou partie de la séquence nucléotidique de SEQ ID NO:1 comme définie dans le premier objet selon l'invention, codant pour tout ou partie d'une enzyme impliquée dans la synthèse des caroténoïdes, représentée par SEQ ID NO:2, précédé par une origine de réplication de la transcription des plantes, de manière à ce que le vecteur puisse générer de l'ARNm dans les cellules des plantes.

L'invention peut donc être utilisée pour modifier la synthèse des caroténoïdes, par exemple augmenter ou diminuer, voire arrêter, la production des couleurs associées à la déshydrogénation du phytoène. Par exemple, l'inhibition de la couleur rouge dans les fruits tels que les tomates, par transformation avec un vecteur comprenant une séquence anti-sens, donne un fruit d'une couleur attrayante se rapprochant du jaune, comme celle de certains poivrons. Il existe déjà des tomates jaunes de cette sorte, mais la présente invention fournit un moyen de transférer la caractéristique couleur dans des lignées, sans qu'un programme de reproduction prolongé ne soit nécessaire et puisse de ce fait engendrer l'altération d'autres caractéristiques de la plante.

L'augmentation de la synthèse des caroténoïdes, par transformation avec un vecteur comprenant une séquence sens, peut permettre de produire des tomates de couleur plus rouge, ce qui peut apparaître plus appétissant au consommateur. L'invention peut également servir à introduire une couleur rouge à l'intérieur d'une plante, ailleurs que dans le fruit. L'augmentation de la synthèse des caroténoïdes dans une plante peut être effectuée en insérant une ou plusieurs copies fonctionnelles du gène d'ADN complémentaire, ou le gène complet, sous contrôle d'un promoteur fonctionnel dans les cellules de plantes.

Les vecteurs de transformation des plantes pour diminuer ou arrêter la synthèse des caroténoïdes, c'est à dire les vecteurs anti-sens, peuvent être très courts. Dans un mode de réalisation préférentiel, on choisira des séquences de bases homologues ayant une longueur d'au moins 10 bases. Il n'existe pas de limite supérieure théorique à la séquence de bases, elle peut être aussi longue que l'ARNm produit par la plante. Dans un mode de réalisation très préférentiel, on utilisera cependant des séquences de longueur comprise entre 100 et 1000 bases.

On sait que les plantes mutantes chez qui le gène OTBC est inactif présentent un aspect panaché, les plantes sont vertes et blanches. On propose une application de la stratégie anti-sens, qui vise à éliminer la production d'ARNm et donc de la protéine OTBC, qui viserait à produire des plantes au feuillage panaché comme par exemple des plantes ornementales, de type Nicotiana ou Pétunia ou toute autre plante ornementale, qui se prête à la transformation génétique et qui pourrait recevoir une construction anti-sens dans le but d'empêcher la production de la protéine OTBC.

Les produits de recombinaison d'ADN peuvent être fabriqués en utilisant des techniques standards. Par exemple, la séquence d'ADN à transcrire peut être obtenue en traitant un vecteur contenant ladite séquence avec des enzymes de restriction pour couper le segment approprié. La séquence d'ADN de transcription peut également être engendrée en cyclisant et liant des oligonucléotides synthétiques ou en utilisant des oligonucléotides synthétiques dans une PCR (« Polymerase Chain Reaction ») pour engendrer des sites de restriction à chaque extrémité. La séquence d'ADN est alors clonée à l'intérieur d'un vecteur contenant une séquence promotrice d'initiation et une séquence de terminaison. Si l'on désire obtenir une séquence d'ADN anti-sens, le clonage sera effectué de manière à ce que la séquence d'ADN coupée soit inversée par rapport à son orientation dans le brin duquel elle a été coupée.

Dans un produit de recombinaison exprimant un ARN anti-sens, le brin qui était initialement le brin matrice devient le brin codant, et vice versa. Le produit de recombinaison transcrira de ce fait un ARNm dont la séquence de base est complémentaire de tout ou partie de la séquence de l'ARNm de l'enzyme. De ce fait, les deux brins d'ARN sont complémentaires non seulement dans leurs séquences de bases mais également dans leur orientation (5' vers 3').

Dans un produit de recombinaison exprimant un ARN sens, la matrice et les brins transcrits gardent l'orientation du gène initial de la plante. Les produits de recombinaison exprimant de l'ARN sens transcrivent un ARNm ayant une séquence de bases qui est homologue en tout ou partie avec la séquence de l'ARNm. Dans les produits de recombinaison exprimant l'enzyme fonctionnelle, la région codante complète du gène est reliée à des séquences de contrôle de la transcription capables de s'exprimer dans la plante.

Par exemple, les produits de recombinaison selon la présente invention peuvent être fabriqués comme décrit ci-après. Un vecteur adapté contenant la séquence de base souhaitée pour la transcription, tel que notamment un clone d'ADN complémentaire d'OTBC, est traité avec des enzymes de restriction pour couper la séquence. On clone alors l'ADN ainsi obtenu, dans une orientation inversée si on le souhaite, dans un second vecteur contenant la séquence promotrice souhaitée et la séquence de terminaison souhaitée. Parmi les promoteurs adaptés, on peut citer le promoteur nommé 35S du virus du CaMV, à titre d'exemple de promoteur considéré comme étant constitutif ; le promoteur du gène de la polygalacturonase de tomate (voir Bird et al., 1998, Plant Molecular Biology, 11:651-662) comme exemple de promoteur impliqué dans la régulation des fruits ; ou encore le promoteur du gène de la petite sous-unité de la ribulose bis-phosphate carboxylase, comme exemple de promoteur exprimé dans les tissus verts. Les séquences de terminaison comprennent le terminateur NOS du gène nopaline synthase.

Il peut être intéressant de modifier l'activité enzymatique de la plante durant seulement le développement et/ou le mûrissement des fruits. L'utilisation d'un promoteur constitutif tendra à modifier le taux et l'activité des enzymes dans toutes les parties de la plante transformée, alors que l'utilisation d'un promoteur spécifique à un tissu contrôlera de manière plus sélective l'expression du gène et modifiera l'activité, par exemple la coloration des fruits. De ce fait, en mettant en oeuvre l'invention, par exemple dans des poivrons, il sera adapté d'utiliser un promoteur qui permettra l'expression spécifique au cours du développement et/ou le mûrissement des fruits. Enfin, l'ARN sens ou anti-sens sera alors produit seulement dans les organes de la plante où l'on souhaite qu'il y ait une action. Parmi les promoteurs spécifiques de développement et ou de mûrissement des fruits qui peuvent être utilisés, on peut citer le promoteur de stimulation de la polygalacturonase (Demande de brevet internationale publiée sous le numéro WO-A-92/08798), le promoteur E8 (Dieckman & Fiscer, 1998, EMBO, 7:3315-3320) et le promoteur spécifique des fruits 2A11 (Pear et al., 1989, Plant Molecular biology, 13: 639-651).

Un sixième objet selon l'invention concerne une cellule de plante transformée par un vecteur défini dans le cinquième objet selon l'invention.

L'homme de l'art dans la technique du génie génétique végétal connaît bien aujourd'hui les différentes techniques d'obtention de plantes génétiquement modifiées. On sait que la paroi végétale constitue une barrière mécanique naturelle particulièrement efficace à la pénétration de tout matériel étranger dans la cellule et, en particulier, à celle d'ADN. Les différents techniques spécifiques d'introduction de l'ADN dans la cellule végétale sont par exemple l'utilisation de la bactérie *Agrobacterium tumefaciens,* l'électroporation de protoplastes, la micro-injection d'ADN nu, l'utilisation de canon à particules ou biolistique, ou la transformation de protoplastes.

Afin de pouvoir sélectionner les cellules effectivement transformées, on introduit, en plus du gène codant pour le caractère que l'on recherche, un gène marqueur. On choisira préférentiellement un gène conférant une résistance à un antibiotique. Les cellules sont alors sélectionnées par culture sur un milieu contenant cet antibiotique. Seules les cellules possédant le gène de résistance pourront se multiplier. La présence du gène d'intérêt peut également être vérifiée par hybridation avec de l'ADN complémentaire de l'ADN introduit.

Le produit de recombinaison selon l'invention est transféré à l'intérieur d'une cellule de plante cible. La cellule de plante cible peut être une partie d'une plante complète ou peut être une cellule isolée ou partie d'un tissu qui peut être régénéré à l'intérieur d'une plante complète. La cellule de plante cible peut être choisie parmi toute espèce de plante monocotylédone ou dicotylédone. Les plantes adaptées comprennent toute plante portant des fruits, telles que notamment les tomates, les mangues, les pêches, les pommes, les poires, les fraises, les bananes, les melons, les poivrons, les piments, le paprika, les plantes ayant des feuilles, des fleurs ou tout autre organe dans lesquels on souhaite modifier le contenu en caroténoïdes.

Les produits de recombinaison selon l'invention peuvent être utilisés pour transformer toute plante, en utilisant toute technique adaptée pour transformer des plantes selon l'invention. Les cellules des plantes monocotylédones et dicotylédones peuvent être transformées de différentes manières connues par l'homme de l'art. Dans la plupart des cas, les cellules de ces plantes, particulièrement lorsque ce sont des cellules de plantes dicotylédones, peuvent être mise en culture pour générer une plante entière qui se reproduit par la suite en engendrant des générations successives de plantes modifiées génétiquement. Tout procédé adapté pour la transformation des plantes peut être utilisé. Par exemple, les plantes dicotylédones, telles que la tomate et le melon, peuvent être transformées en utilisant le plasmide Ti *Agrobacterium.* De telles plantes transformées peuvent se reproduire par croisement, ou par culture de cellule ou de tissu.

Un septième objet selon l'invention concerne une plante, ou fragment de plante, particulièrement fruit, graine, pétale, feuille, comprenant des cellules définies selon le sixième objet de l'invention.

Les plantes ou fragments de plantes, génétiquement modifiées selon l'invention avec un vecteur comprenant une séquence sens, notamment pour augmenter la production de caroténoïdes, comprennent un taux élevé en précurseur de la vitamine A par rapport au taux normal produit par la plante.

Les caroténoïdes, outre leur rôle dans la couleur de la plante, ont également un rôle de protection des plantes contre les dommages que peut produire une haute intensité lumineuse. De ce fait, les plantes, contenant par modification génétique un taux plus élevé de ces caroténoïdes, peuvent présenter un grand intérêt pour les régions où la culture s'effectue avec des changements de température importants.

Les plantes modifiées génétiquement peuvent présenter des couleurs différentes, selon que l'on ait augmenté ou diminué la synthèse des caroténoïdes. Plus particulièrement, les produits de recombinaison de l'OTBC peuvent être utilisés pour stimuler ou inhiber la production des couleurs associées aux caroténoïdes produits lors des réactions de désaturation, par exemple le lycopène rouge, ou dérivés de produits comme la couleur jaune/orange associée au béta-carotène. La stimulation de la production des béta-carotènes, avec un produit de recombinaison sens de sur-expression, peut permettre de produire des poivrons de couleur jaune/orange, ou bien une couleur déterminée par un dérivé des béta-carotènes telle qu'un rouge plus intense, du fait de la biosynthèse de capsorubine ou de capsanthine. Les poivrons obtenus s'avéreront plus appétissants par le consommateur.

Comme exemple de plantes génétiquement modifiées selon la présente invention, on citera plus particulièrement les plantes portant des fruits. Les fruits de ces plantes peuvent donc être rendus plus attirants pour le consommateur, en stimulant ou intensifiant à l'intérieur une couleur spécifique. Comme autres plantes qui peuvent être modifiées génétiquement, on peut citer les tubercules tels que les radis, les navets et les pommes de terre, de même que les céréales tels que le maïs, le blé, l'orge et le riz.

Les plantes modifiées génétiquement selon l'invention, peuvent également contenir d'autres produits de recombinaison, par exemple des produits de recombinaison ayant d'autres effets, notamment sur le mûrissement des fruits. Par exemple, des fruits ayant une couleur plus intense, modifiés selon la présente invention, peuvent également contenir des produits de recombinaison, soit qui inhibent la production de certaines enzymes telles que la polygalacturonase et la pectinestérase, soit qui interfèrent avec la production d'éthylène. Les fruits qui contiennent ces deux types de produits de recombinaison peuvent être engendrés, soit par des transformations successives, soit en croisant deux variétés qui contiennent chacune un des produits de recombinaison, puis en sélectionnant parmi la descendance ceux qui contiennent les deux produits de recombinaison.

Un huitième objet selon l'invention concerne un procédé pour modifier la production de caroténoïdes dans une plante, soit en augmentant la production de caroténoides, soit en abaissant ou inhibant la production de caroténoïdes par la plante, relativement au contenu normal de caroténoïdes produits par la plante, ledit procédé comprenant la transformation de cellules desdites plantes à transformer avec un vecteur défini dans le cinquième objet selon l'invention.

Un neuvième objet selon l'invention concerne un procédé pour produire des caroténoïdes dans une cellule de plante, ou eucaryote ou procaryote, ledit procédé comprenant la transformation de cellules desdites plantes, des cellules eucaryotes ou procaryotes à transformer avec un vecteur défini dans le cinquième objet selon l'invention.

Les bêta-carotènes, produits par un organisme eucaryote ou procaryote exprimant un produit de recombinaison codant pour l'enzyme OTBC, peuvent être extraits pour être utilisés en tant que colorant, antioxydant ou précurseur de la vitamine A.

Enfin, l'invention concerne aussi un procédé pour la sélection de composé présentant un caractère herbicide, selon lequel on met en contact ledit agent avec des cellules ou des membranes de cellules, notamment des cellules de l'invention, et on observe une diminution de la consommation d'oxygène par les membranes desdites cellules, liée à l'inhibition de l'oxydase terminale associée à la biosynthèse des caroténoïdes. Des techniques appropriées pour effectuer cette observation sont notamment illustrée dans l'exemple 6.

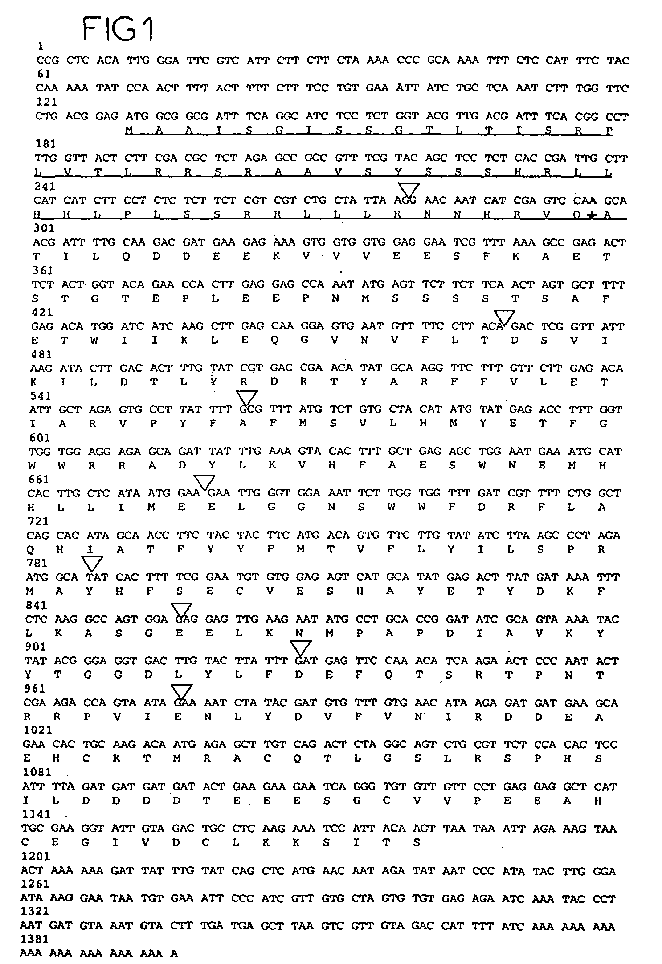

La figure 1 présente la séquence d'ADNc et la séquence d'acides aminés correspondante de l'OTBC. Le peptide N-terminal de transit potentiel du chloroplaste est souligné. Le point probable de clivage est indiqué par une étoile (*). Les triangles ouverts indiquent la position des introns.

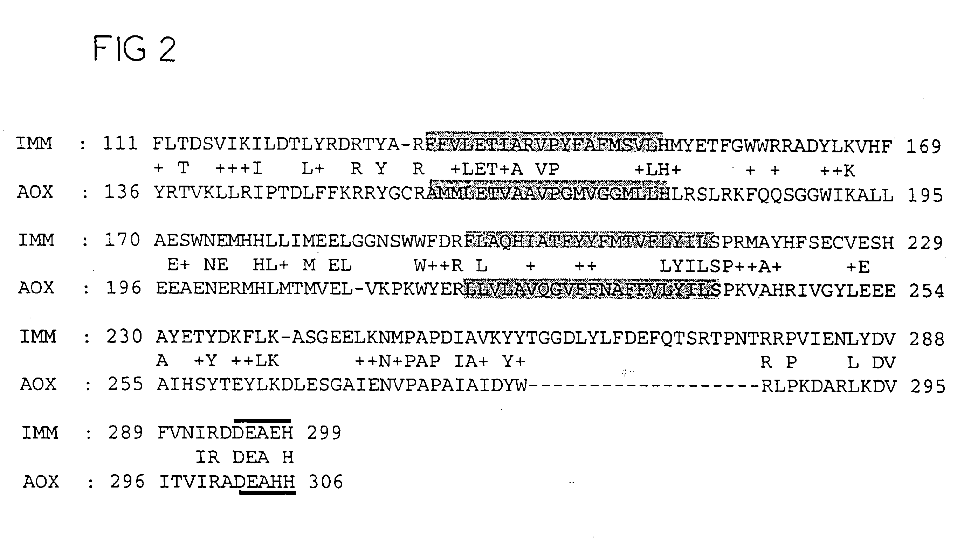

La figure 2 montre la comparaison entre la protéine OTBC et la protéine AOX de la graine de soja. (+) indique les acides aminés similaires. Les acides aminés présentés dans une boite font partie des domaines en hélice trans-membranaire prédits. Les motifs de liaison du fer sont surlignés.

La figure 3 présente l'alignement des séquences en acides aminés pour la tomate (T), le poivron (P) et Arabidopsis (A) et la séquence consensus. Dans cette séquence consensus, les acides aminés conservés sont indiqués en lettre majuscule et les acides aminés relativement conservés sont indiqués en lettre minuscule.

La figure 4 représente la consommation d'oxygène dans des membranes cellulaires de *E.coli* isolées pour des cellules témoins transformées par un vecteur de clonage de l'invention et pour des cellules exprimant le produit du gène « IMMUTANS » (plastid terminal oxidase).

### Exemple 1 : Détail du clonage du locus codant pour la protéine OTBC

### 1- Isolement du mutant.

On a provoqué la mutation par l'utilisation d'un transposon introduit dans le génome de la plante *Arabidopsis thaliana cultivar landsberg-erecta.*

Cette technique est largement décrite dans un article (Long, D., Martin, M., Sundberg, E., Swinburne, J., Puangsomlee P., and Coupland, G. (1993) The maize transposable element system Ac/ Ds as a mutagen in Arabidosis : Identification of an albino mutation induced by Ds insertion. Pro. Natl. Science USA, 10, 10370-10374) et a été mise en oeuvre par d'autres au laboratoire de George Coupland au John Innes Centre for Plant Science, Colney, Norwich, NR4 7UH, Nordwich, Grande-Bretagne.

La transposition de l'élément transposable Dissociator (Ds) utilisé ici a été déclenchée par la production de la protéine transposase (ou Transposase de l'élément Activator, Ac).

Parmi la descendance d'une plante ayant subi la transposition de l'élément Ds, on a identifié plusieurs plantes à l'aspect mutant albinos, différent du sauvage par l'absence de pigmentation verte (chlorophylle). On a également identifié des plantes d'aspect sauvage mais qui transmettent la mutation à leur descendance. Ces plantes sont identifiées comme hétérozygotes, portant la mutation sur un seul chromosome. Les plantes homozygotes ont un phénotype mutant et portent la mutation sur les deux chromosomes homologues.

### 2- Test de liaison de la mutation à l'élément transposable Ds.

Cette expérience a été faite dans le but de prouver que la mutation observée est causée par l'insertion de l'élément Ds dans un gène nécessaire au bon fonctionnement de la plante et à son aspect sauvage.

L'élément transposable, ou transposon, Ds, est construit de façon a porter un gène de résistance a l'antibiotique hygromycine (décrit dans les références précédentes). On a fait pousser la descendance de 35 plantes hétérozygotes qui portent la mutation albinos sur un milieu gélosé contenant une dose létale d'hygromycine, toutes les plantes qui portent la mutation sont aussi résistantes à l'hygromycine. On en tire la conclusion que la mutation est liée au gène de résistance porté par le transposon.

On a isolé une portion d'ADN de plante résistante à l'antibiotique hygromycine, jouxtant le transposon. Ceci a été fait selon la méthode IPCR ou PCR-inverse décrite dans les références précédentes.

Par expérience dite de « Southern blot », on a remarqué que les lignées qui portent la mutation ont une altération de l'ADN génomique. Cette altération est révélée lorsque l'on utilise comme « sonde » la portion d'ADN isolée jouxtant le transposon.

### 3- Isolement du gène

On a, en utilisant une méthode de criblage de banque d'ADN génomique, isolé un clone contenant un fragment d'ADN génomique pouvant contenir la version sauvage inaltérée du gène interrompu chez le mutant.

La banque d'ADN criblée a été construite. Elle est décrite dans la publication de Whitelam, G.C., Johnson, E., Peng, J., Carol P., Anderson, M.L., Cowl, J.S. & Harberd, N.P. (1993) Phytochrome A null mutants of Arabidopsis display a wild-type phenotype in white light. The Plant Cell 5, 757-768.

On a déterminé la séquence totale d'un fragment de restriction obtenu par digestion enzymatique du clone d'ADN génomique par l'enzyme EcoR I. La séquence obtenue couvre 3000 paires de bases. Parmi ces 3000 paires de bases, on trouve une partie identique à la séquence du fragment bordure préalablement isolé, confirmant l'identité entre l'ADN isolé et le gène interrompu par le transposon.

### 4- Isolement et caractérisation de la séquence codante.

On a utilisé une banque d'ADNc, qui est une banque commerciale vendue par CLONTECH Laboratories, Inc.. Il s'agit d'une banque d'ADNc faite à partir d'ARNm extraits d'*Arabidopsis thaliana,* transformés en ADNc, puis clonés dans le vecteur plasmidique pGAD10.

A partir de cette banque de données d'ADNc, et selon les techniques habituelles, utilisant le gène identifié précédemment comme sonde, on a isolé plusieurs clones contenant un ADNc d'une taille d'environ 1400 paires de bases.

On a déterminé la séquence totale de l'ADNc et montré que celui-ci est entièrement compris dans le fragment d'ADN génomique préalablement identifié. On a placé la partie codante (ou exons) et la partie non codante (introns) du gène sur la séquence du gène. Le gène porte 9 exons et 8 introns. L'insertion du transposon Ds est identifiée au début du deuxième exon et vient donc interrompre la partie codante du gène.

La séquence de l'ADNc présente un codon de départ potentiel suivi par un cadre de lecture ouvert de 350 acides aminés, codant pour une protéine potentielle de 39 kDa dénommé OTBC. Une recherche d'homologie en utilisant le programme blastp [(Altshul et al. (1997), Gapped BLAST and PSI-BLAST : a new generation of protein database search programs Nucleics Acids Res. **25**, 3389-3402] a révélé une homologie faible mais significative avec des polypeptides appartenant à la famille des protéines oxydase alternative ou terminale oxydase de mitochondries (AOX). Aucune autre homologie significative n'a été trouvée. L'homologie commence à l'acide aminé 111 et présente 29% d'identité (45% de similarité) avec l'oxydase de soja. Malgré la faible identité avec la protéine AOX, une recherche par ordinateur de structures secondaires et des domaines potentiels de la signification biologique ont révélé une similarité structurale entre la protéine OTBC et AOX. Des domaines en hélice trans-membranaire trouvés dans AOX sont situés à des positions similaires sur la séquence peptidique de l'OTBC, suggérant une localisation membranaire de l'OTBC et également une configuration similaire à celle de l'AOX dans la membrane. De plus, un motif de liaison du fer se trouve conservé entre OTBC et AOX. L'alignement des séquences entre les protéines OTBC et AOX montre une insertion de 19 acides aminés dans la protéine OTBC qui correspond à une partie des exons 7 et 8.

La séquence N-terminale de la protéine OTBC présente les caractéristiques d'un peptide de transit du chloroplaste, qui est riche en leucine, arginine et sérine / thréonine. Une analyse par ordinateur du potentiel peptide de transit (psort software, Nakai and Kanehisa, 1992) a suggéré une cible possible de OTBC au niveau des compartiments des thylakoides du chloroplaste.

### 5- Identification de la mutation.

L'aspect du mutant est proche de celui d'un mutant déjà décrit dans la littérature : le mutant "immutans", Wetzel C.M., Jiang C-Z., Meehan L.J., Voytas D.L., Rodermel S.R. (1994) Nuclear-organelle interactions: the immutans variegation mutant of Arabidopsis is plastid autonomous and impaired in carotenoid biosynthesis, Plant Journal 6, 161-175.

On a croisé le mutant « immutans » (allèle spotty, cf. référence précédente) avec celui qui a été isolé selon l'invention. La descendance du croisement est d'aspect mutant, ce qui est un résultat attendu si les deux mutations affectent le même gène. On peut donc affirmer que le gène identifié correspond à la version sauvage du locus IMMUTANS et que le mutant obtenu porte une version interrompue du gène dont le produit est alors inactif.

Le premier objet de la présente invention se distingue donc du mutant ci-dessus, en ce qu'il code pour une protéine présentant une activité enzymatique identique ou équivalente à celle de l'OTBC, alors que le produit codé par « immutans » n'a pas d'activité.

### Exemple 2 : Construction d'un vecteur de l'invention par introduction d'ADNc codant pour l'OTBC de poivron dans un vecteur d'expression de plantes

Le vecteur pBI121 (commercialisé par Clontech laboratories, Inc) est un vecteur adapté à cette construction.

Il comporte une région T-DNA que la bactérie *Agrobacterium tumefaciens* peut transférer dans le génome des plantes.

Cette région T-DNA comporte, entre autres, un promoteur constitutif (le promoteur nommé 35S du virus CaMV), le gène GUS suivi du terminateur NOS (du gène nopaline synthase). Le gène GUS ne présentant aucun intérêt pour l'invention, est remplacé par un ADNc codant pour l'OTBC. Cet ADNc sera donc placé sous le contrôle du promoteur 35S et du terminateur NOS.

Tout autre promoteur constitutif ou non (dans ce dernier cas, il devra être spécifique de l'organe dont on souhaite modifier les propriétés) et tout autre terminateur sont également utilisables.

Un ADNc codant pour l'OTBC a été sous-cloné originellement dans le site de restriction Notl du plasmide bactérien pBluescriptKS : il a ainsi été flanqué de sites de coupures BamHl en 5' et Sacl en 3'.

Cet ADNc est excisé du plasmide pBluescriptKS par les enzymes de restriction BamHl et Sacl. Ce fragment BamHI-Sacl est inséré dans le vecteur pBI121 lui-même coupé par ces enzymes : le site BamHl se trouve en 3' du promoteur 35S et en 5' du gène GUS, le site Sacl se trouve en 3' du gène GUS et en 5' du terminateur NOS.

Après ligation, sont sélectionnés les dérivés du vecteur pBI121 dans lequel l'ADNc codant pour l'OTBC (c'est-à-dire sans intron) a remplacé le gène GUS.

### Exemple 3 : Transformation d'une cellule de plante pour obtenir une cellule transformée de l'invention.

Le vecteur de transformation de plante dérivé de pBI121 obtenu à l'exemple 2 est introduit dans la souche d'Agrobacterium LBA4404 par électroporation. La souche recombinante est sélectionnée en présence de 50 *µ*g/ml de kanamycine.

Cette souche transformée d'Agrobacterium est utilisée pour la transformation de cellules de plantes, par exemple de tabac.

La technique utilisée à cet effet et qui peut être remplacée par toute autre technique de transformation, est celle de l'infection de disques foliaires de plantules de tabac cultivées in vitro. Les cellules transformées de plantes sont sélectionnées en présence de kanamycine. Agrobacterium est éliminé par l'antibiotique céfotaxime. Les disques foliaires sont cultivés sur milieu de culture végétale en présence d'hormones végétales (auxine et cytokinines) favorisant la croissance de cals. Les cals issus de la croissance des cellules transformées sont utilisés pour la régénération de plantes entières par les techniques classiques. Par exemple, les cals sont transférés sur milieu de culture végétale en présence de cytokinine pour induire la formation de pousses. Celles-ci sont ensuite coupées et transférées sur milieu de culture végétale sans hormone afin de régénérer des racines. Les antibiotiques kanamycine (afin de sélectionner la croissance de tissus transformés) et céfotaxime (afin d'éliminer complètement Agrobacterium) sont maintenus pendant toutes ces phases de culture.

Les plantes transformées sont mises en culture stérilement en présence de kanamycine et céfotaxime puis sont transférées en terre et cultivées en serre jusqu'à la récolte des graines. La présence du transgène a été confirmée par hybridation de l'ADN génomique de ces plantes avec une sonde spécifique issue du vecteur de transformation utilisé.

### Exemple 4 : Clonage et caractérisation d'ADNc de fruits de poivron et de tomate correspondant à l'enzyme oxydase terminale associée à la biosynthèse des caroténoïdes (OTBC)

On a utilisé la partie d'ADNc de « immutans » d'Arabidopsis codant pour le peptide OTBC mature comme sonde pour rechercher une banque d'ADNc de poivron vert ou rouge dans des conditions non stringentes. Tous les clones positifs qui ont été analysés, ont semblé dériver du même gène, comme le suggèrent les séquences identiques observées dans la région non traduite en 3'. La séquence d'ADN du clone complet est présentée dans la liste de séquence sous l'identificateur SEQ ID NO :3. La séquence déduite en acides aminés est présentée dans la liste de séquences sous l'identificateur SEQ ID NO :4. L'ADNc de poivron a ensuite été utilisé pour isoler l'ADNc correspondant à partir d'une banque d'ADNc de tomate rouge (SEQ ID NO :5).

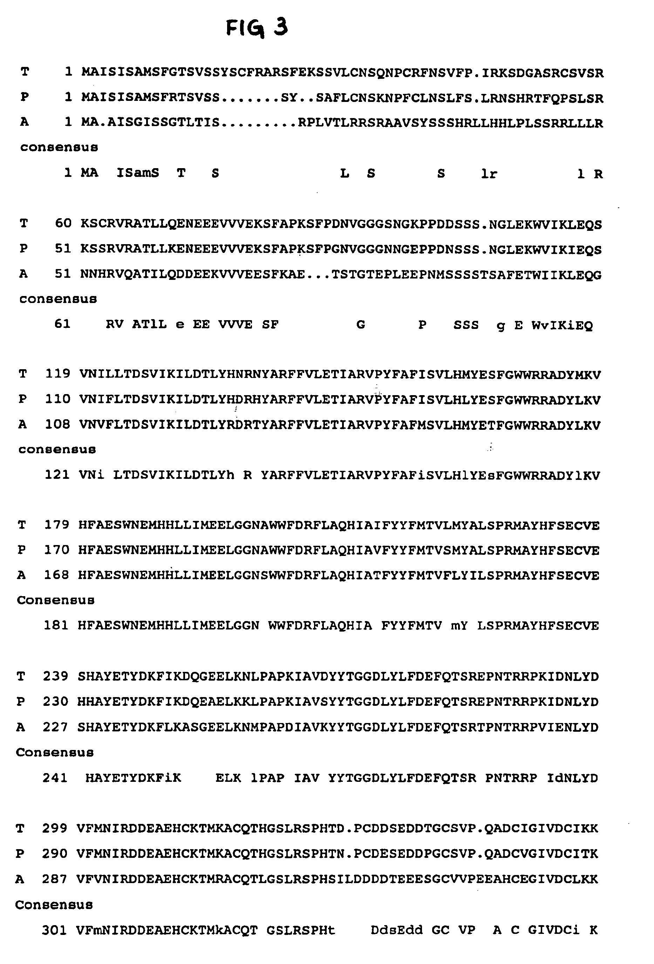

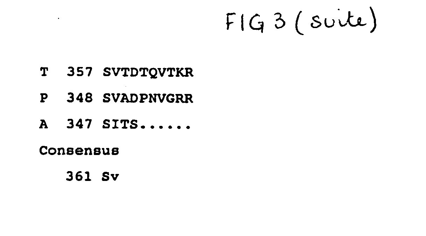

La figure 3 présente la comparaison entre la séquence déduite en acides aminés précitée et les séquences de l'OTBC de poivron et d'Arabidopsis.

Les peptides de transit utilisés pour le ciblage dans les plastides ont révélés une similarité de séquence, à l'exception de la région N-terminale et de la région près du site de clivage supposé (ATR/Q-AT). Les polypeptides d'OTBC mature partagent pourtant une forte similarité de séquence, ce qui signifie qu'ils présentent les mêmes propriétés.

Un alignement des séquences de l'OTBC a en outre révélé la présence de deux domaines trans-membranaires potentiels conservés, séparés par un segment hydrophile fortement conservé. Le domaine N-terminal est essentiellement hydrophile et contient un long segment d'acides aminés faiblement conservés. Le domaine C-terminal est aussi principalement hydrophile et contient un motif (EAEH) conservé qui concorde avec un site putatif de fixation du fer (ExxH). En outre, la région contient 6 résidus cystéine conservés dans l'OTBC, alors que le reste du polypeptide est dépourvu de résidus cystéine.

Certains de ces résidus cystéine peuvent être impliqués dans la dimérisation covalente de la protéine.

### Exemple 5 : Expression des gènes de l'OTBC au cours du mûrissement du fruit chez le poivron et la tomate

Afin de définir le mécanisme d'expression des gènes de l'OTBC, on a extrait l'ARN total du fruit à différents stades du mûrissement. Le mécanisme d'expression a été déterminé par transription inverse de l'ARN total, suivie d'une réaction de polymérisation en chaîne (RT-PCR).

Le gène de l'OTBC est exprimé au cours du développement et du mûrissement du fruit pour le poivron. En outre, il a un mécanisme d'expression similaire à celui des gènes codant pour les caroténoïde-désaturases, c'est-à-dire la phytoène-désaturase et la zéta-carotène-désaturase. On observe une augmentation du taux de transcription entre le stade vert non mûr et le stade vert mûr (fruit d'une taille adulte), suivie d'une autre augmentation entre le stade vert mûr et le stade de dégradation (signes visibles précoces de changement de couleur). Le taux de transcription reste ensuite assez constant (avec une légère diminution au cours de l'étape de rougissement).

Le gène de l'OTBC est également exprimé durant le développement et le mûrissement du fruit chez la tomate. Chez la tomate, il présente aussi un mécanisme d'expression similaire à celui des gènes codant pour les caroténoïde-désaturases (phytoène-désaturase et zéta-carotène-désaturase). On observe une augmentation du taux de transcription entre le stade vert non mûr et le stade vert mûr (fruit d'une taille adulte), suivie d'une autre augmentation plus forte entre le stade vert mûr et le stade de dégradation.

Lorsque l'empreinte de la protéine des fruits du poivron et de la tomate a été recherchée, en utilisant des anticorps dirigés contre l'OTBC, ce polypeptide a été retrouvé à différents stades de développement du fruit. Ces essais ont mis en évidence une augmentation du taux de la protéine OTBC, depuis le stade vert mûr vers le stade de dégradation. Ce taux de protéine est resté élevé au cours du mûrissement du fruit.

Ces résultats démontrent que les gènes de l'OTBC sont exprimés et que la protéine OTBC est présente dans le fruit. De manière analogue aux enzymes structurales intervenant dans la désaturation des caroténoïdes, le gène de l'OTBC est induit et les protéines sont accumulées au cours du mûrissement quand la biosynthèse des caroténoïdes est accrue.

Les résultats exposés dans la description mettent en évidence que l'OTBC est un élément du système de la biosynthèse des caroténoïdes.

On peut envisager d'utiliser la protéine OTBC pour modifier la biosynthèse des caroténoïdes, notamment dans les tissus ou les cellules végétales ou dans les bactéries présentant un système de biosynthèse des caroténoïdes peu efficace ou inefficace. L'OTBC pourrait être produite en même temps que les enzymes structurales de la biosynthèse des caroténoïdes pour accroître l'efficacité de la production des caroténoïdes.

### Exemple 6: Propriétés catalytiques de l'OTBC analysées après son expression chez E. coli

Un produit de synthèse consistant en la région codant pour le polypeptide mature OTBC de *Arabidopsis* a été inséré dans un vecteur d'expression procaryote (tel que pQE31, commercialisé par QIAGEN, étant entendu que tout autre vecteur conduirait à des résultats identiques).

La région codante destinée à être insérée dans le vecteur d'expression peut être obtenue par clivage à l'aide d'enzymes de restriction intervenant près des codons correspondant au site de clivage du peptide de transit.

Alternativement, une amplification par PCR de la région codante peut être effectuée. Les oligonucléotides suivants seront avantageusement utilisés pour l'amplification de la séquence de l'OTBC d'Arabidopsis :
5'-GCAACGATTTTGCAAGACG-3' et
5'-TTAACTTGTAATGGATTTCTTGAG-3'

D'autres produits d'assemblage comportant la région codante pour l'OTBC chez d'autres espèces (comme le poivron ou la tomate) peuvent aussi être employés.

Ces plasmides peuvent être introduits dans des cellules de *E.coli* selon des techniques classiques. Afin d'obtenir la protéine recombinante chez *E.coli*, les cellules sont cultivées dans les conditions suivantes : 10 ml d'une pré-culture d'une nuit complète dans un milieu riche sont déposées dans 300 ml de milieu M9 (Na₂HPO₄ 34mM, KH₂PO₄ 22mM, NH₄Cl 18 mM, NaCl 8,5 mM, MgSO₄ 1 mM, CaCl₂ 0,1 mM, thiamine 1 mM) contenant 0,2% de glycérol et l'apport en antibiotique nécessaire pour stopper la croissance des cellules qui ont perdu le plasmide. Le développement des bactéries est poursuivi à 37°C sous agitation vigoureuse jusqu'à la phase de développement mi-exponentielle, de préférence jusqu'à lecture d'une densité optique de 0,3 à 600 nm.

Après induction de ce gène chimère par l'inducteur IPTG et addition de FeSO₄ à 1 mg/l, la culture est entretenue à 25°C sous agitation vigoureuse pendant 3 heures. Les cellules sont ensuite recueillies par centrifugation à 4°C, lavées avec du MgCl₂ 10 mM, du saccharose 0,75 M, du Tris-HCl 20 mM, à pH 7,5, et de nouveau centrifugées. Les cellules sont ensuite mises en suspension dans du saccharose 0,75 M, du Tris-HCl 20 mM, à pH 7,5, et lysées par addition de lysozyme (0,2 mg/ml) et de EDTA (25 mM) à 30°C pendant 30 minutes, puis soumises à un choc osmotique par addition de deux volumes d'eau, puis traitées aux ultrasons à 0°C. Une centrifugation standard dans une centrifugeuse à vitesse lente permet d'éliminer les cellules non lysées et les débris. Une centrifugation à grande vitesse (par exemple dans un rotor 50Ti de Beckman à 40 000 tours / min) à 4°C conduit à l'obtention d'une membrane qui est mise en suspension dans du saccharose 0,75 M, Tris-HCl 20 mM, à pH 7,5, et maintenue à 4°C.

Pour tester l'activité enzymatique de l'OTBC, la consommation d'oxygène par les membranes résultantes est mesurée à l'aide d'une électrode à oxygène standard et est exprimée en nmoles d'O₂ consommé par minute et par gramme de protéine.

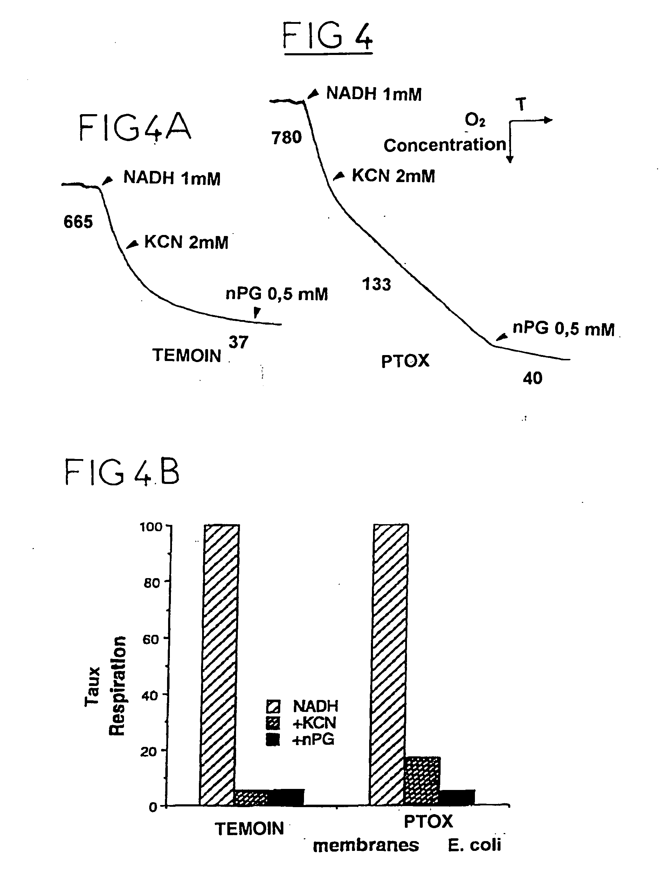

Comme le montre la Figure 4, l'addition de NADH provoque la consommation d'oxygène à la fois dans la membrane témoin (transformée par le vecteur de clonage) et dans la membrane contenant l'OTBC. Cette consommation d'oxygène augmente lorsque l'on ajoute de la plastoquinone 0,2 mM. L'addition de KCN inhibe fortement la consommation d'oxygène dans les membranes témoins. Dans les membranes contenant l'OTBC, on observe une consommation d'oxygène résistant au cyanure élevée. Ceci reflète l'activité oxydoréductase plastoquinol : oxygène de l'OTBC, laquelle activité peut être inhibée par addition de gallate de n-propyle (nPG) 0,5 mM. L'addition de nPG (0,5 mM) à la membrane témoin avant le KCN ne produit pas d'effet indiquant que le composé n'interfère pas avec le flux normal d'électrons dans les membranes de *E.coli* (Figure 4).

Ce test peut être utilisé pour étudier le pouvoir inhibiteur d'un composé sur l'activité de l'OTBC. Ainsi un inhibiteur peut être contrôlé quand il n'a pas d'effet sur la chaîne respiratoire endogène de *E.coli,* en particulier sur le complexe I de la chaîne qui oxyde NADH. Néanmoins, si tel est le cas, NADH peut être substitué par le succinate en tant que donneur d'électrons sans passer par le complexe I. Tout inhibiteur de l'activité de l'OTBC peut être testé sur des plants appropriés, par arrosage du sol, addition d'un milieu de culture et dépôt direct sur les feuilles, vis-à-vis de l'inhibition de la biosynthèse des caroténoïdes entraînant un blanchiment et alors trouver une application comme herbicide.

L'essai décrit peut être modifié pour réaliser un criblage à grande échelle d'inhibiteurs de l'activité de l'OTBC, et leur application en tant qu'herbicide. Dans ce cas, la mesure de la consommation d'oxygène à l'aide d'une électrode à oxygène sera de préférence remplacée par une autre méthode de mesure.

L'activité oxydase de l'OTBC peut être déterminée en mesurant la consommation de NADH au cours de la réaction, par exemple, par spectrophotométrie, en mesurant l'absorbance à 340 nm. La consommation de NADH et la production de NAD au cours de l'essai doit entraîner une diminution de l'absorbance à 340 nm. Alternativement, on peut utiliser toute coloration spécifique de NAD ou de NADH pour contrôler les changements de NAD ou de NADH pendant l'essai.

Si l'on utilise le succinate comme donneur d'électrons, dans l'essai, l'activité respiratoire des membranes bactériennes conduira à l'oxydation du succinate en fumarate. Alors l'activité de l'OTBC pourra être suivie en présence de KCN, en mesurant les concentrations en succinate et fumarate qui évoluent au cours de l'essai.

Selon une autre possibilité, on peut utiliser un donneur d'électrons artificiel. Le Phéazine Méto-sulfate (PMS) en est un exemple. Il peut être oxydé par la succinate déshydrogénase des membranes bactériennes ; il est incolore à l'état réduit et est coloré en jaune à l'état oxydé.

Des échantillons de membrane bactérienne contenant l'OTBC vont oxyder le PMS en présence de KCN. Un inhibiteur de l'activité de l'OTBC empêchera l'apparition de la couleur jaune due à l'oxydation du PMS. Ce test de mise en oeuvre simple peut être conduit dans des boîtes multi-puits permettant de réaliser un dépistage en masse de molécules capables d'inhiber l'activité de l'OTBC.

## Revendications

1. Séquence d'ADN, comprenant au moins une région codante constituée par :
- la séquence nucléotidique représentée par SEQ ID NO : 1, transcrivant un ARNm, cet ARNm codant pour l'enzyme Oxydase Terminale associée à la Biosynthèse des Caroténoïdes (OTBC) décrite par SEQ ID NO :2, ou son complémentaire,
- toute séquence nucléotidique modifiée :
présentant, par rapport à SEQ ID NO : 1, une ou des modifications choisies parmi les additions, les suppressions et les substitutions de nucléotides,
présentant avec SEQ ID NO : 1 au moins 70% d'identité,
et transcrivant un ARNm qui code lui-même pour l'OTBC décrite par SEQ ID NO : 2 ou une protéine modifiée de ladite OTBC ayant une activité enzymatique équivalente à celle de l'OTBC décrite par SEQ ID NO :2, ou son complémentaire.

2. Séquence selon la revendication 1, **caractérisée en ce qu'**elle comprend au moins une région codante constituée par toute séquence nucléotidique modifiée :
présentant, par rapport à SEQ ID NO : 1, une ou des modifications choisies parmi les additions, les suppressions et les substitutions de nucléotides,
présentant avec SEQ ID NO : 1 au moins 80% d'identité,
et transcrivant un ARNm qui code lui-même pour l'OTBC décrite par SEQ ID NO : 2 ou une protéine modifiée de ladite OTBC ayant une activité enzymatique équivalente à celle de l'OTBC décrite par SEQ ID NO :2, ou son complémentaire.

3. Séquence selon la revendication 1, **caractérisée en ce qu'**elle comprend au moins une région codante constituée par une séquence nucléotidique transcrivant un ARNm qui code pour une protéine décrite par SEQ ID NO : 4.

4. Séquence selon l'une quelconque des revendications 1 à 3, **caractérisée en ce que** ladite séquence nucléotidique est choisie parmi SEQ ID NO : 3 et SEQ ID NO : 5.

5. ARNm transcrit à partir d'une séquence selon la revendication 1, ou à partir de son complémentaire.

6. Protéine ayant une activité enzymatique, Oxydase Terminale associée à la Biosynthèse des Caroténoïdes, codée par une séquence selon l'une quelconque des revendications 1 à 4.

7. Protéine selon la revendication 6, **caractérisée en ce qu'**elle est choisie parmi SEQ ID NO : 2, SEQ ID NO : 4 et la séquence codée par SEQ ID NO : 5.

8. ADN recombiné **caractérisé en ce qu'**il comprend une séquence d'ADN selon l'une quelconque des revendications 1 à 4, ladite séquence étant insérée dans une séquence hétérologue, lesdites séquences transcrivant tout ou partie d'une séquence d'ARNm codant pour tout ou partie de l'enzyme OTBC, cette dernière ayant une activité enzymatique équivalente à l'enzyme OTBC de la plante.

9. ADN recombiné selon la revendication 8, **caractérisé en ce qu'**il comprend les éléments nécessaires pour contrôler l'expression de la séquence nucléotidique insérée, particulièrement une séquence promotrice et une séquence de terminaison de transcription.

10. Vecteur de la transformation de plantes, adapté pour augmenter la biosynthèse des caroténoïdes, comprenant tout ou partie de la séquence nucléotidique SEQ ID NO : 1 selon la revendication 1, codant pour tout ou partie d'une enzyme impliquée dans la synthèse des caroténoïdes, décrite par SEQ ID NO : 2, précédé par une origine de réplication de la transcription des plantes, de manière à ce que le vecteur puisse générer de l'ARNm dans les cellules des plantes.

11. Cellule de plante transformée par un vecteur selon la revendication 10.

12. Plante ou fragment de plante, particulièrement fruit, graine, pétale, feuille, comprenant des cellules selon la revendication 11.

13. Procédé pour modifier la production des caroténoïdes dans une plante, soit en augmentant la production de caroténoïdes, soit en abaissant ou inhibant la production de caroténoïdes par la plante, relativement au contenu normal de caroténoïdes produits par la plante, ledit procédé comprenant la transformation de cellules desdites plantes à transformer avec un vecteur selon la revendication 10.

14. Procédé pour produire des caroténoïdes dans une cellule de plante, ou eucaryote ou procaryote, ledit procédé comprenant la transformation de cellules desdites plantes, des cellules eucaryotes ou procaryotes à transformer avec un vecteur selon la revendication 10.

15. Procédé pour la sélection de composés comprenant un caractère herbicide, selon lequel on met en contact ledit agent avec des cellules ou des membranes de cellules de la revendication 11, et on observe une diminution de la consommation d'oxygène par les membranes desdites cellules, liée à l'inhibition de l'oxydase terminale associée à la biosynthèse des caroténoïdes.

## Claims

1. DNA sequence comprising at least one coding region consisting of:
- the nucleotide sequence represented by SEQ ID NO: 1 transcribing an mRNA, this mRNA encoding the TOCB (Terminal Oxidase associated with Carotenoid Biosynthesis) enzyme described by SEQ ID NO: 2, or its complement,
- any modified nucleotide sequence:
exhibiting, relative to SEQ ID NO: 1, one or more modifications chosen from nucleotide additions, deletions and substitutions,
exhibiting at least 70% identity with SEQ ID NO: 1,
and transcribing an mRNA which itself encodes the TOCB described by SEQ ID NO: 2 or a modified protein of said TOCB having an enzymatic activity which is equivalent to that of the TOCB described by SEQ ID NO: 2, or its complement.

2. Sequence according to Claim 1, **characterised in that** it comprises at least one coding region composed of any modified nucleotide sequence:
exhibiting, relative to SEQ ID NO: 1, one or more modifications chosen from nucleotide additions, deletions and substitutions,
exhibiting at least 80% identity with SEQ ID NO: 1,
and transcribing an mRNA which itself encodes the TOCB described by SEQ ID NO: 2 or a modified protein of said TOCB having an enzymatic activity which is equivalent to that of the TOCB described by SEQ ID NO: 2, or its complement.

3. Sequence according to Claim 1, **characterized in that** it comprises at least one coding region composed of a nucleotide sequence transcribing an mRNA which codes for a protein described by SEQ ID NO: 4.

4. Sequence according to any one of Claims 1 to 3, **characterized in that** the said nucleotide sequence is selected from SEQ ID NO: 3 and SEQ ID NO: 5.

5. mRNA transcribed from a sequence according to Claim 1, or from its complement.

6. Protein having a Terminal Oxidase associated with Carotenoid Biosynthesis enzymatic activity encoded by a sequence according to any one of Claims 1 to 4.

7. Protein according to Claim 6, **characterized in that** it is selected from SEQ ID NO: 2, SEQ ID NO: 4 and the sequence encoded by SEQ ID NO: 5.

8. Recombinant DNA, **characterized in that** it comprises a DNA sequence according to any one of Claims 1 to 4, said sequence being inserted into a heterologous sequence, said sequences transcribing all or a portion of a sequence of an mRNA encoding all or a portion of the TOCB enzyme, said enzyne having enzymatic activity equivalent to that of the TOCB enzyme of the plant.

9. Recombinant DNA according to Claim 8, **characterized in that** it comprises the elements required to control the expression of the inserted nucleotide sequence, particularly a promoter sequence and a transcription termination sequence.

10. Plant transformation vector which is adapted to increase carotenoid biosynthesis, comprising all or a portion of the nucleotide sequence SEQ ID NO: 1 according to Claim 1, encoding all or a portion of an enzyme involved in carotenoid synthesis, represented by SEQ ID NO: 2, preceded by an origin of replication of the transcription of the plants, such that the vector can generate mRNA in the plant cells.

11. Plant cell transformed with a vector according to Claim 10.

12. Plant, or plant fragment, particularly a fruit, seed, petal or leaf, comprising cells according to Claim 11.

13. Process for modifying the production of carotenoids in a plant, either by increasing the production of carotenoids, or by reducing or inhibiting the production of carotenoids by the plant, relative to the normal content of carotenoids produced by the plant, said process comprising the transformation of cells of said plants to be transformed with a vector according to Claim 10.

14. Process for producing carotenoids in a plant cell, or eukaryotic or prokaryotic cell, said process comprising the transformation of cells of said plants, eukaryotic or prokaryotic cells to be transformed with a vector according to Claim 10.

15. Process for selecting compounds comprising a herbicidal character, in which said agent is placed in contact with cells or cell membranes of Claim 11, and a reduction in the consumption of oxygen by the membranes of said cells, which is associated with the inhibition of the terminal oxidase associated with carotenoid biosynthesis, is observed.

## Patentansprüche

1. DNA-Sequenz, enthaltend mindestens eine codierende Region, die aus Folgendem besteht:
- der durch SEQ ID Nr. 1 dargestellten Nukleotidsequenz, die in eine mRNA transkribiert wird, wobei diese mRNA für das durch SEQ ID Nr. 2 beschriebene Enzym terminale Oxidase codiert, das mit der Biosynthese von Carotinoiden zusammenhängt (OTBC), oder der dazu komplementären Sequenz,
- jeder modifizierten Nukleotidsequenz,
die in Bezug auf SEQ ID Nr. 1 eine oder mehrere Modifikationen aufweist, die aus Additionen, Suppressionen und Substitutionen von Mukleotiden ausgewählt sind,
die mindestens 70% Identität zu SEQ ID Nr. 1 aufweist
und in eine mRNA transkribiert wird, die selbst für die durch SEQ ID Nr. 2 beschriebene OTBC oder ein modifiziertes Protein dieser OTBC mit einer enzymatischen Aktivität codiert, die zu derjenigen der durch SEQ ID Nr. 2 beschriebenen OTBC äquivalent ist, oder der dazu komplementären Sequenz.

2. Sequenz nach Anspruch 1, **dadurch gekennzeichnet, dass** sie mindestens eine codierende Region umfasst, die aus jeder modifizierten Nukleotidsequenz besteht:
die in Bezug auf SEQ ID Nr. 1 eine oder mehrere Modifikationen aufweist, die aus Additionen, Suppressionen und Substitutionen von Nukleotiden ausgewählt sind,
die mindestens 80% Identität zu SEQ ID Nr. 1 aufweist
und in eine mRNA transkribiert wird, die selbst für die durch SEQ ID Nr. 2 beschriebene OTBC oder ein modifiziertes Protein dieser OTBC mit einer enzymatischen Aktivität codiert, die zu derjenigen der durch SEQ ID Nr. 2 beschriebenen OTBC äquivalent ist, oder der dazu komplementären Sequenz.

3. Sequenz nach Anspruch 1, **dadurch** gekernzeichnet, dass sie mindestens eine codierende Region umfasst, die aus einer Nuklcotidsequenz bestcht, die in eine mRNA transkribiert wird, die ein durch SEQ ID Nr. 4 beschriebenes Protein codiert.

4. Sequenz nach einem der Ansprüche 1 bis 3, **dadurch gekennzeichnet, dass** diese Nukleotidsequenz aus SEQ ID Nr. 3 und SEQ 10 Nr. 5 ausgewählt ist.

5. mRNA, die von einer Sequenz nach Anspruch 1 oder von der dazu komplementären Sequenz transkribiert wird.

6. Protein mit enzymatischer Aktivität, terminale Oxidase, die mit der Biosynthese der Carotinoide zusammenhängt, codiert von einer Sequenz nach einem der Ansprüche 1 bis 4.

7. Protein nach Anspruch 6, **dadurch gekennzeichnet, dass** es aus SEQ ID Nr. 2, SEQ ID Nr. 4 und der von SEQ ID Nr. 5 codierten Sequenz ausgewählt ist.

8. Rekombinante DNA, **dadurch gekennzeichnet, dass** sie eine DNA-Sequenz nach einem der Ansprüche 1 bis 4 enthält, wobei diese Sequenz in eine heterologe Sequenz inseriert ist, wobei diese Sequenzen in eine gesamte oder einen Teil einer mRNA-Sequenz trinskribiert werden, die für das gesamte oder einen Teil des Enzyms OTBC codiert, wobei dieses Letztere eine enzymatische Aktivität hat, die zu derjenigen von Pflanzen-OTBC äquivalent ist.

9. Rekombinante DNA nach Anspruch 8, **dadurch gekennzeichnet, dass** sie die notwendigen Elemente zur Steuerung der Expression der inserierten Nukleotidsequanz enthält, insbesondere eine Promotorsequenz und eine Transkriptionsterminationsseguenz.

10. Pflanzentransformationsvektor, der dafür ausgelegt ist, die Biosynthese von Carotinoiden zu erhöhen, enthaltend die gesamte oder einen Teil 1 der Nukleotidsequenz SEQ ID Nr. 1 nach Anspruch 1, die für das gesamte oder einen Teil eines an der Carotinoidsynthese beteiligten, durch SEQ ID Nr. 2 beschriebenen Enzyms codiert, der ein Replikationsursorung der Transkription in Pflanzen vorausgeht, so dass der Vektor die mRNA in den Zellen der Pflanzen erzeugen kann.

11. Pflanzenzelle, die mit einem Vektor nach Anspruch 10 transformiert ist.

12. Pflanze oder Pflanzenteil, insbesondere Frucht, Korn, Kronblatt, Blatt, die/das die Zellen nach Anspruch 11 enthält.

13. Verfahren zur Modifikation der Produktion von Carotinoiden in einer Pflanze entweder durch Erhöhen oder durch Senken oder Hemmen der Produktion von Carotinoiden durch die Pflanze relativ zum normalen Gehalt an Carotinoiden, die von der Pflanze produziert werden, wobei das Verfahren die Transformation von Zellen dieser zu transformierenden Pflanzen mit einem Vektor nach Anspruch 10 umfasst.

14. Verfahren zur Produktion von Carotinoiden entweder in einer eukaryotischen oder einer prokaryotischen Pflanzenzelle, wobei das Verfahren die Transformation von zu transformierenden Zellen dieser Pflanzen, eukaryotischen oder prokaryotischen Zellen, mit einem Vektor nach Anspruch 10 umfasst.

15. Verfahren zur Selektion von Verbindungen mit herbizider Eigenschaft, gemäß dem man dieses Mittel mit den Zellen oder den Membranen von Zellen nach Anspruch 11 in Kontakt bringt und eine Verminderung des Sauerstoffverbrauchs durch die Membranen dieser Zellen aufgrund der Hemmung der terminalen Oxidase, die mit der Carotinoidbiosynthese zusammenhängt, beobeichtet.


### LISTE DE SEQUENCES

<110> UNIVERSITE JOSEPH FOURIER
<120> Séquence d'ADNc transcrivant un ARNm codant pour l'oxydase terminale associée à la biosynthèse des caroténoïdes et utilisations
<130> OTBC
<140>
   <141>
<150> FR9813283
   <151> 1998-10-20
<160> 5
<170> PatentIn Ver. 2.1
<210> 1
   <211> 1396
   <212> ADN
   <213> Arabidopsis thaliana
<400> 1
   ccgctcacat tgggattcgt cattcttctt ctaaaacccg caaaatttct ccatttctac 60
   caaaaatatc caacttttac ttttctttcc tgtgaaatta tctgctcaaa tctttggttc 120
   ctgacggaga tggcggcgat ttcaggcatc tcctctggta cgttgacgat ttcacggcct 180
   ttggttactc ttcgacgctc tagagccgcc gtttcgtaca gctcctctca ccgattgctt 240
   catcatcttc ctctctcttc tcgtcgtctg ctattaagga acaatcatcg agtccaagca 300
   acgattttgc aagacgatga agagaaagtg gtggtggagg aatcgtttaa agccgagact 360
   tctactggta cagaaccact tgaggagcca aatatgagtt cttcttcaac tagtgctttt 420
   gagacatgga tcatcaagct tgagcaagga gtgaatgttt tccttacaga ctcggttatt 480
   aagatacttg acactttgta tcgtgaccga acatatgcaa ggttctttgt tcttgagaca 540
   attgctagag tgccttattt tgcgtttatg tctgtgctac atatgtatga gacctttggt 600
   tggtggagga gagcagatta tttgaaagta cactttgctg agagctggaa tgaaatgcat 660
   cacttgctca taatggaaga attgggtgga aattcttggt ggtttgatcg ttttctggct 720
   cagcacatag caaccttcta ctacttcatg acagtgttct tgtatatctt aagccctaga 780
   atggcatatc acttttcgga atgtgtggag agtcatgcat atgagactta tgataaattt 840
   ctcaaggcca gtggagagga gttgaagaat atgcctgcac cggatatcgc agtaaaatac 900
   tatacgggag gtgacttgta cttatttgat gagttccaaa catcaagaac tcccaatact 960
   cgaagaccag taatagaaaa tctatacgat gtgtttgtga acataagaga tgatgaagca 1020
   gaacactgca agacaatgag agcttgtcag actctaggca gtctgcgttc tccacactcc 1080
   attttagatg atgatgatac tgaagaagaa tcagggtgtg ttgttcctga ggaggctcat 1140
   tgcgaaggta ttgtagactg cctcaagaaa tccattacaa gttaataaat tagaaagtaa 1200
   actaaaaaag attatttgta tcagctcatg aacaatagat ataatcccat atacttggga 1260
   ataaaggaat aatgtgaaat tcccatcgtt gtgctagtgt gtgagagaat caaataccct 1320
   aatgatgtaa atgtactttg atgagcttaa gtcgttgtag accattttat caaaaaaaaa 1380
   aaaaaaaaaa aaaaaa 1396
<210> 2
   <211> 351
   <212> PRT
   <213> Arabidopsis thaliana
<400> 2
   Met Ala Ala Ile Ser Gly Ile Ser Ser Gly Thr Leu Thr Ile Ser Arg
    1               5                  10                  15
   Pro Leu Val Thr Leu Arg Arg Ser Arg Ala Ala Val Ser Tyr Ser Ser
               20                  25                  30
   Ser His Arg Leu Leu His His Leu Pro Leu Ser Ser Arg Arg Leu Leu
           35                  40                  45
   Leu Arg Asn Asn His Arg Val Gln Ala Thr Ile Leu Gln Asp Asp Glu
       50                  55                  60
   Glu Lys Val Val Val Glu Glu Ser Phe Lys Ala Glu Thr Ser Thr Gly
   65                  70                  75                  80
   Thr Glu Pro Leu Glu Glu Pro Asn Met Ser Ser Ser Ser Thr Ser Ala
                   85                  90                  95
   Phe Glu Thr Trp Ile Ile Lys Leu Glu Gln Gly Val Asn Val Phe Leu
               100                 105                 110
   Thr Asp Ser Val Ile Lys Ile Leu Asp Thr Leu Tyr Arg Asp Arg Thr
           115                 120                 125
   Tyr Ala Arg Phe Phe Val Leu Glu Thr Ile Ala Arg Val Pro Tyr Phe
       130                 135                 140
   Ala Phe Met Ser Val Leu His Met Tyr Glu Thr Phe Gly Trp Trp Arg
   145                 150                 155                 160
   Arg Ala Asp Tyr Leu Lys Val His Phe Ala Glu Ser Trp Asn Glu Met
                   165                 170                 175
   His His Leu Leu Ile Met Glu Glu Leu Gly Gly Asn Ser Trp Trp Phe
               180                 185                 190
   Asp Arg Phe Leu Ala Gln His Ile Ala Thr Phe Tyr Tyr Phe Met Thr
           195                 200                 205
   Val Phe Leu Tyr Ile Leu Ser Pro Arg Met Ala Tyr His Phe Ser Glu
   210 215 220
   Cys Val Glu Ser His Ala Tyr Glu Thr Tyr Asp Lys Phe Leu Lys Ala
   225 230 235 240
   Ser Gly Glu Glu Leu Lys Asn Met Pro Ala Pro Asp Ile Ala Val Lys
   245 250 255
   Tyr Tyr Thr Gly Gly Asp Leu Tyr Leu Phe Asp Glu Phe Gln Thr Ser
   260 265 270
   Arg Thr Pro Asn Thr Arg Arg Pro Val Ile Glu Asn Leu Tyr Asp Val
   275 280 285
   Phe Val Asn Ile Arg Asp Asp Glu Ala Glu His Cys Lys Thr Met Arg
   290 295 300
   Ala Cys Gln Thr Leu Gly Ser Leu Arg Ser Pro His Ser Ile Leu Asp
   305 310 315 320
   Asp Asp Asp Thr Glu Glu Glu Ser Gly Cys Val Val Pro Glu Glu Ala
   325 330 335
   His Cys Glu Gly Ile Val Asp Cys Leu Lys Lys Ser Ile Thr Ser
   340 345 350
<210> 3
   <211> 1387
   <212> ADN
   <213> poivron
<400> 3
   ccacgcgtcc gataaaaaaa tcaagaatgg cgatttccat atctgctatg agttttcgaa 60
   cttcagtttc ttcttcatat tcagcatttt tgtgcaattc caagaaccca ttttgtttga 120
   attctctatt ttcacttagg aattctcata gaacttttca gccttcgtta tcaaggaaat 180
   caagtagagt tcgagcaacg ttgttaaaag agaatgaaga agaagtggtt gtggagaaat 240
   cttttgcacc taagagtttt cctggtaatg tgggaggggg aaataatggg gagccacccg 300
   ataattcatc ctcgaacggt ctggagaaat gggttataaa gattgagcag tctgtaaata 360
   tctttctcac ggattcagtg ataaagattc ttgacacttt gtatcacgac cgacactatg 420
   cgaggttttt cgttctggaa acaattgcaa gagttcctta ttttgcattt atatctgttc 480
   ttcacttgta cgagagcttt ggttggtgga gacgagcaga ttatctgaag gtgcattttg 540
   ccgagagctg gaatgagatg caccatttac tcattatgga ggaattaggt ggaaatgctt 600
   ggtggtttga ccgattcctt gcgcaacata ttgctgtatt ctattatttc atgacagtct 660
   cgatgtatgc tttgagcccg agaatggcat atcatttctc tgaatgtgtg gagcaccatg 720
   catacgagac ttacgataaa ttcatcaagg atcaagaagc ggaattgaag aaattgcccg 780
   ctccaaagat tgcagtgagc tactacaccg gaggtgactt gtatttgttc gatgagtttc 840
   aaacatcacg agagcctaat actcgaaggc caaaaataga taatctgtac gacgtattca 900
   tgaacatcag agatgacgaa gcagagcatt gtaagacaat gaaagcgtgt caaacccatg 960
   ggagcctccg ctcccctcac acaaatccat gcgatgagtc tgaagacgat ccaggttgtt 1020
   cagtgcctca ggccgattgt gtaggtatcg tggattgtat aacgaaatct gtcgctgatc 1080
   ctaacgtcgg cagaaggtag ggaaaggaaa aacgcagaac gaaactatac atgtatatac 1140
   cagtacagcc aaatatacaa gaaatataca tacatattgt atcttttact ctctgaggaa 1200
   gagcttgtca aattgcccaa aaaatgggta ggcacttggt tttgttttca cctttcaata 1260
   atttgtacta aactatgaac aaatttgctc cggcacacta caactccata ggggtcctgt 1320
   tacgcttctg aactaaattt taacatattt ttgtcaacct tctcagcaaa aaaaaaaaaa 1380
   aaaaaaa 1387
<210> 4
   <211> 357
   <212> PRT
   <213> poivron
<400> 4
   Met Ala Ile Ser Ile Ser Ala Met Ser Phe Arg Thr Ser Val Ser Ser
    1               5                  10                  15
   Ser Tyr Ser Ala Phe Leu Cys Asn Ser Lys Asn Pro Phe Cys Leu Asn
               20                  25                  30
   Ser Leu Phe Ser Leu Arg Asn Ser His Arg Thr Phe Gln Pro Ser Leu
           35                  40                  45
   Ser Arg Lys Ser Ser Arg Val Arg Ala Thr Leu Leu Lys Glu Asn Glu
       50                  55                  60
   Glu Glu Val Val Val Glu Lys Ser Phe Ala Pro Lys Ser Phe Pro Gly
   65                  70                  75                  80
   Asn Val Gly Gly Gly Asn Asn Gly Glu Pro Pro Asp Asn Ser Ser Ser
                   85                  90                  95
   Asn Gly Leu Glu Lys Trp Val Ile Lys Ile Glu Gln Ser Val Asn Ile
               100                 105                 110
   Phe Leu Thr Asp Ser Val Ile Lys Ile Leu Asp Thr Leu Tyr His Asp
           115                 120                 125
   Arg His Tyr Ala Arg Phe Phe Val Leu Glu Thr Ile Ala Arg Val Pro
       130                 135                 140
   Tyr Phe Ala Phe Ile Ser Val Leu His Leu Tyr Glu Ser Phe Gly Trp
   145 150 155 160
   Trp Arg Arg Ala Asp Tyr Leu Lys Val His Phe Ala Glu Ser Trp Asn
   165 170 175
   Glu Met His His Leu Leu Ile Met Glu Glu Leu Gly Gly Asn Ala Trp
   180 185 190
   Trp Phe Asp Arg Phe Leu Ala Gln His Ile Ala Val Phe Tyr Tyr Phe
   195 200 205
   Met Thr Val Ser Met Tyr Ala Leu Ser Pro Arg Met Ala Tyr His Phe
   210 215 220
   Ser Glu Cys Val Glu His His Ala Tyr Glu Thr Tyr Asp Lys Phe Ile
   225 230 235 240
   Lys Asp Gln Glu Ala Glu Leu Lys Lys Leu Pro Ala Pro Lys Ile Ala
   245 250 255
   Val Ser Tyr Tyr Thr Gly Gly Asp Leu Tyr Leu Phe Asp Glu Phe Gln
   260 265 270
   Thr Ser Arg Glu Pro Asn Thr Arg Arg Pro Lys Ile Asp Asn Leu Tyr
   275 280 285
   Asp Val Phe Met Asn Ile Arg Asp Asp Glu Ala Glu His Cys Lys Thr
   290 295 300
   Met Lys Ala Cys Gln Thr His Gly Ser Leu Arg Ser Pro His Thr Asn
   305 310 315 320
   Pro Cys Asp Glu Ser Glu Asp Asp Pro Gly Cys Ser Val Pro Gln Ala
   325 330 335
   Asp Cys Val Gly Ile Val Asp Cys Ile Thr Lys Ser Val Ala Asp Pro
   340 345 350
   Asn Val Gly Arg Arg
   355
<210> 5
   <211> 1284
   <212> ADN
   <213> tomate
<400> 5
   gaattcggca cgagcggcac gagcagaaaa ctaacaactt tcccactttg gaattttctt 60
   taccttacct aagaagggta ttaatttgat tcttgtggga aggaagaagg atcaagaatg 120
   gcgatttcga tttctgctat gagttttgga acctcagttt cttcatattc ttgttttaga 180
   gctaggagtt ttgagaagtc atcagtttta tgcaattccc agaacccatg tcggtttaat 240
   tctgtttttc cgattcggaa atctgatggg gcttcacggt gttctgtttc taggaaatca 300
   tgtagagttc gagcaacgtt gttacaagag aatgaagaag aagtggttgt ggagaaatct 360
   tttgcaccta agagttttcc tgataacgtg ggagggggaa gtaatgggaa gccaccagat 420
   gattcatcct ctaacggtct agagaaatgg gttataaagc ttgagcagtc tgtaaatatc 480
   ttactcacgg attcagtgat aaagattctt gacactttgt atcacaaccg aaactatgcg 540
   aggtttttgg ttctggaaac aattgcaagg gttccttatt ttgcatttat atcggttctt 600
   cacatgtatg agagctttgg ctggtggaga agggcagatt atatgaaagt gcattttgct 660
   gaaagctgga atgagatgca ccatttgctc attatggaag aattaggggg aaatgcttgg 720
   tggtttgatc gatttcttgc acaacatata gctatattct attatttcat gacagtcttg 780
   atgtatgctt tgagcccgag aatggcatat catttctctg aatgtgtgga gagccatgca 840
   tacgagactt acgataaatt catcaaggat caaggagagg aattgaagaa tttgcccgct 900
   ccaaagattg cagtggacta ctacacggga ggtgacttat atttatttga tgagtttcaa 960
   acttcacgag agcctaatac tcgaagacca aaaatagata atctctatga cgtattcatg 1020
   aacattagag atgacgaagc agagcattgt aaaacgatga aagcctgtca aactcacggg 1080
   agccttcgtt ctccacacac agatccatgc gatgattctg aagatgatac agggtgttcc 1140
   gtacctcaag ctgattgtat aggtatcgtg gattgtataa agaagtcagt caccgatact 1200
   caagtaacca aaaggtagga aaaggaaaaa cgcggacaaa ctatacttgt atatactagt 1260
   atagacaaaa aaaaaaaaaa aaaa                                        1284